# EUROPEAN PATENT SPECIFICATION

(11) **EP 3 887 812 B1**
(45) Date of publication and mention of the grant of the patent: **07.06.2023**
(21) Application number: 19816925.2
(22) Date of filing: 28.11.2019
(51) Int. Cl.: G01N 27/22, A61B 5/00, A61F 13/42

(54) **A SENSOR, SYSTEM AND METHOD FOR DETECTING OR SENSING MOISTURE OR WETNESS OF AN ARTICLE**
SENSOR, SYSTEM UND VERFAHREN ZUR ERKENNUNG ODER ERFASSUNG VON FEUCHTIGKEIT ODER NÄSSE EINES ARTIKELS
CAPTEUR, SYSTÈME ET PROCÉDÉ DE DÉTECTION DE L'HUMIDITÉ D'UN ARTICLE

(30) Priority: 28.11.2018 NZ 18748811
(43) Date of publication of application: 06.10.2021
(60) Divisional application: 23153760.6
(73) Proprietor: Tilkoblede Belgium BVBA, 1702 Dilbeek (BE)
(72) Inventor: VAN DE SANDE, Benoit, 1703 Schepdaal (BE); VAN DE SANDE, Bram, 1703 Schepdaal (BE)
(74) Representative: Mewburn Ellis LLP
(86) International application number: PCT/IB2019/060269
(87) International publication number: WO 2020/110057

(56) References cited:
- WO-A1-2015/184148
- WO-A2-2009/084942
- US-A1- 2014 026 653
- US-A1- 2014 291 677

## Description

### TECHNICAL FIELD

The present disclosure according to claim 1 relates to a system for detecting or sensing moisture or wetness of an article to which the system is attached.

### BACKGROUND

Many different types of articles are used in a typical household. During use of these articles they may come into contact with a liquid such as water and or aqueous solutions or mixtures. The presence of the liquid may not be immediately observable, and so timely and adequate actions in response cannot be determined.

The article must then be constantly checked to determine the presence of water.

An example of such an article is an absorbent article, in particular a disposable personal care article, such as a nappy, a diaper, a baby pant, an adult incontinent garment, and the like. Once the nappy has been fitted there is no way to tell whether liquid (such as urine) is present in the nappy.

A further example may be a piece of clothing for example a layer in a multi layered outfit. The outfit may have an external waterproof layer, with multiple under layers to ensure the wearer is kept warm. If the waterproof layer is breached an outer layer of the under layers is exposed to the elements, the outer layer may become wet. Because of the other intermediate layers between the wearer and the outer layer, the breach of the waterproof layer may not be immediately apparent to the wearer leading to further water ingress.

Chemically activated indicators are available which contain various chemical compounds which undergo a chemical reaction when exposed to moisture. These indicators are generally single use and may not indicate the amount of moisture present.

Indicators containing sensors are also available, utilising capacitive, resistive, inductive, optical and sonar sensors. US 2014/026653 A1 relates to capacitive sensors utilized for gas (including humidity) concentration measurements. WO 2009/084942 A2 relates to a transducer comprising a planar capacitor for measuring moisture and humidity content. US 2014/291677 A1 relates to the fabrication of application-specific integrated circuits that incorporate thin film environmental sensors for temperature, pressure, and humidity. WO2015/184148 A1 relates to a heated capacitor and a method of forming the heated capacitor which removes moisture from a moisture-sensitive insulating layer.

### SUMMARY OF THE INVENTION

The invention is defined by the appended claims.

A technical advantage of the present invention is achieved by measuring the ionisation of the environment around the sensor accurately by using the sensor configuration (having a first and second plate) as described.

A further technical advantage may be achieved as the present arrangement does not require full discharge of the electromechanical cell between measurements as is required with capacitive measurement techniques.

### BRIEF DESCRIPTION OF THE DRAWINGS

Fig. 1 shows a bottom view of a sensor.
Fig. 2 shows a top view of a sensor.
Figs. 3 to 5 show cross sections of a sensor.
Fig. 6 shows an example output of a sensor.
Fig. 7 shows top and bottom views of a system including a sensor.
Fig. 8 shows a system attached to an article.
Fig. 9 shows a circuit of the sensor/system.
Fig. 10 shows a flow diagram of a measurement cycle for the system.
Fig. 11 shows a diagrammatic overview of the system.
Fig. 12 shows a graph of measurement readings taken from a sensor at different moisture or wetness levels, and other associated data points.
Fig. 13 shows a flow diagram of a method using 1^{st} and 2^{nd} derivatives.
Fig. 14 shows a series of output graphs (200, 300, 400) from an exemplary sensor wherein series 200 is raw sensor output, and series 300 is a filtered output, and series 400 is a first derivative output.
Fig 15A shows a schematic illustration front view (looking at second side 22) of a sensor, having an array of sub sensors 10.
Fig 15B shows a schematic illustration back to view (looking at first side 21) of a sensor, having an array of sub sensors 10.
Fig 15C shows a schematic cross section view of the sensor of figure 15 A.
Fig 16A shows a schematic illustration of a further alternative sensor 10 configuration.
Fig 16B shows a schematic cross section view of the sensor of Fig. 16A, along line AA.
Fig 16C shows a schematic cross section view of the sensor of Fig. 16A, along line BB.

### DETAILED DESCRIPTION

Disclosed is a sensor, system including the sensor, and method for detecting or sensing moisture or wetness of an article to which the sensor is attached.

The sensor may be configured to measure the presence of any liquid or fluid. For example, in the case where the article is a nappy for a child, the sensor may be configured to measure the presence of urine.

The sensor 10 may be a sensor 10 which has an output which is indicative of the moisture or wetness of an article 28.

In particular, it will be appreciated that the sensor is preferably capable of sensing moisture or wetness of an article without being directly in contact with the moisture or wetness. That is, the sensor is capable of sensing the moisture or wetness within a nappy (for example), when attached to the outside (dry side) of the nappy.

This feature is highly desirable as it allows any one or more of the following advantages:
- ease of adding/removing sensor from article
- ease of access to sensor (particularly useful where sensor module provides visual and/or audible output, or requires other interaction and/or servicing such as charging, re-charging, downloading data etc)
- keep sensor hygienically clean, which makes the apparatus more user-friendly and/or more easily re-usable.

The sensor 10 may be a capacitive type sensor which varies in capacitance depending on the moisture or wetness content of the article 28. For example, an article with a relatively higher moisture content would lead to a relatively higher capacitance of the sensor 10 as compared to an article with a relatively lower moisture content.

In some embodiments, the sensor may be a sensor for which the sensor's charge storage capacity changes depending on the moisture or wetness content of the article 28. For example, an article with a relatively higher moisture content would lead to a relatively higher charge storage capacity of the sensor 10 as compared to an article with a relatively lower moisture content.

In some embodiments the sensor may be an inductive type sensor, and the inductance may vary based on the moisture content of the article.

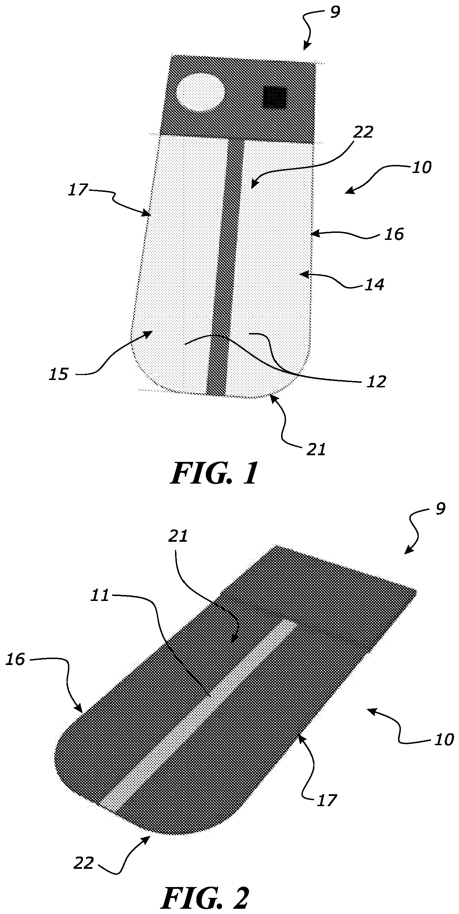

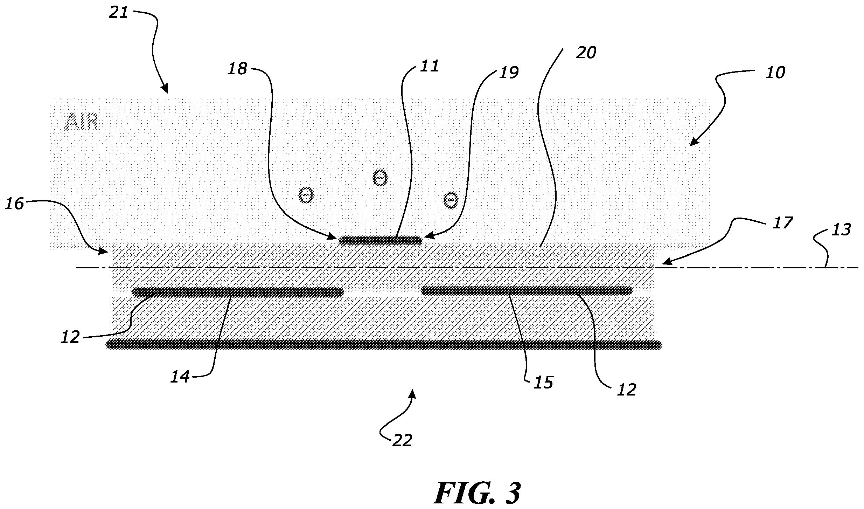

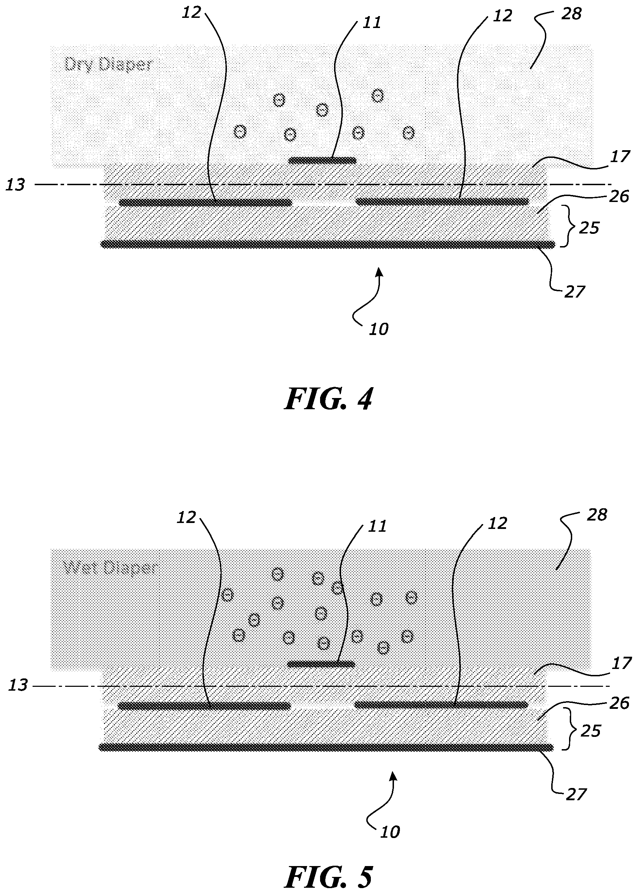

Figs. 1 to 5 show a sensor 10. The sensor may comprise a first plate 11 and a second plate 12. The first plate 11 and the second plate 12 may be electrically conductive.

The first plate 11 and second plate 12 may be made of a metallic material such as copper, or aluminium, or tin, or lead or any conductive metal, or alloy.

The first plate 11 and second plate 12 may be oriented along and/or substantially parallel to a nominal sensor plane 13. The sensor plane 13 may be oriented, in use, substantially parallel to the article 28 to which the sensor 10 is attached.

The first plate 11 may be located between a first portion 14 of the second plate 12, and a second portion 15 of the second plate 12 (when viewed in plan view).

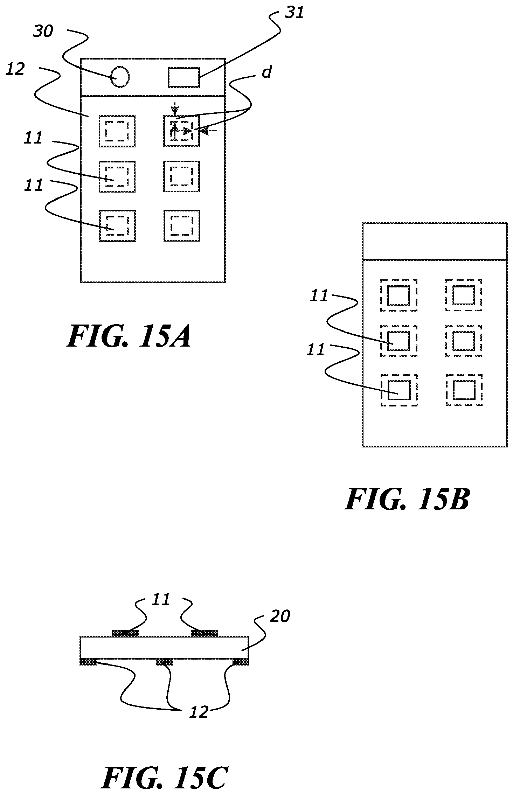

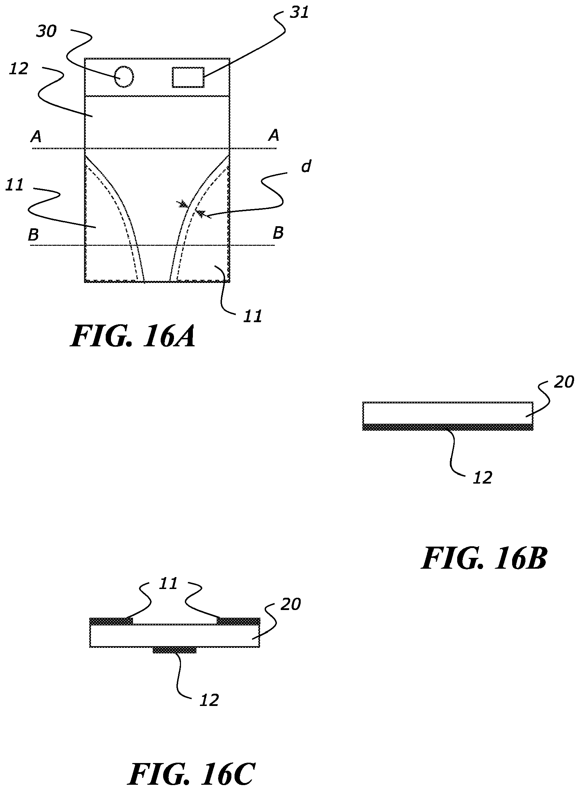

The first portion 14 of the second plate 12 may be located at or adjacent a first edge 18 of the first plate 11, or alternatively near a first edge 18 of the first plate 11 such that no overlap occurs, or such that less than approximately 5% (of the area of the second plate) of overlap occurs when viewed in plan view. Alternatively still, a small gap (equivalent to 'd' in Figs. 15 and 16) may be present.

The second portion 15 of the second plate 12 may be located at or adjacent a second edge 19 of the first plate 11, or alternatively near a second edge 19 of the first plate 11 such that no overlap occurs, or such that less than approximately 5% (of the area of the second plate) of overlap occurs when viewed in plan view. Alternatively still, a small gap (equivalent to 'd' in Figs. 15 and 16) may be present.

The first portion 14 of the second plate 12 may be located at or adjacent a first edge of the sensor, or alternatively near a first edge of the sensor such that no overlap occurs, or such that less than approximately 5% (of the area of the second plate) of overlap occurs when viewed in plan view. Alternatively still, a small gap (equivalent to 'd' in Figs. 15 and 16) may be present.

The second portion 15 of the second plate 12 may be located at or adjacent a second edge of the sensor 10, or alternatively near a second edge of the sensor such that no overlap occurs, or such that less than approximately 5% (of the area of the second plate) of overlap occurs when viewed in plan view. Alternatively still, a small gap (equivalent to 'd' in Figs. 15 and 16) may be present.

The first edge of the sensor 10 may be located adjacent the second edge and/or the first edge of the sensor 10 may be located opposite the second edge.

The first edge 18 of the first plate 11 may be located adjacent the second edge 19 and/or the first edge 18 of the first plate 11 may be located opposite the second edge 19.

Each portion (for example the first portion 14 and the second portion 15) of the second plate 12 may be located on each side of the first plate 11.

The second plate 12 may comprise one or more further portions. The one or more further potions may be located at or adjacent one or more edges of the first plate 11.

The first plate 11 may be or comprise an elongate strip.

The second plate 12 may comprise a pair of elongate strips. Each elongate strip of the pair of elongate strips may be one of the first portion 14 or the second portion 15 of the second plate 12.

In preferred embodiments, when seen in a projection on a plane including the second plate, the shape of the projection of the first plate on the plane is substantially complimentary with the shape of the second plate.

The first plate 11 may be for example a X or + shape which each portion of the second plate 12 being located between adjacent arms or projections of the first plate 11.

For example, the sensor may be configured as an array of sub sensors (equivalent to sensor 10), in order to provide output from separate zones. Such a configuration is shown schematically in Fig. 15, including a battery 30 and controller/IC/measurement means 31.

Fig. 15A shows a second side of a sensor, and illustrates a gap 'd' between the first plate 11 and the second plate 12, such that when viewed in a projection on a plane including the second plate 12 the projection of the first plate does not overlap with the second plate.

Fig. 15B shows a first side of the sensor 10 of Fig. 15A. Fig. 15C shows a cross-section view illustrating a preferred offset through the thickness of sensor 10 between the first plate(s) 11, and the second plate(s) 12.

If present, such zone output data may be used to derive further useful information such as directionality and/or migration of moisture or wetness between/across zones. Alternatively, this could be achieved with the use of multiple sensors.

Fig. 16 schematically illustrates yet another alternative configuration of sensor 10. In this alternative configuration, the second plate 12 tapers towards one edge of sensor 10. In a similar manner to that described above with reference to Fig. 15, this embodiment illustrates a gap 'd' such that when viewed in a plan view, there is no overlap between first plate 11, and second plate 12. Alternatively, when viewed in plan view the gap 'd' may approach zero, or alternatively still a small overlap may represent up to approximately 5% of the area of the second plate 12.

Fig.16B schematically illustrates a cross section along line AA, while Fig. 16C schematically illustrates a cross section along line BB.

The first plate 11 and/or the second plate 12 may comprise at least one angled section.

The second plate 12 may be about 50mm long, and each portion of the second plate, or the second plate may be about 15mm or about 30mm wide.

The first plate 11 may be about 50mm long, and about 5mm wide.

It is anticipated that the external shape of the sensor 10 may take a number of forms. For example, the sensor may be approximately round, elongate rectangle, elongate oval, droplet shaped and/or any other practical variant. In the most preferred embodiments, the thickness of the sensor is small in order for the sensor to generally form a flexible strip appropriate for attachment to an article.

It will be appreciated that various regions of the sensor module may vary in dimension, particularly in sections where additional electronics such as the power supply, PCB, IC controller, communications module etc may be present.

The first plate 11 may be in the same plate as the second plate 12.

The first plate 11 may be vertically offset from the second plate 12. The first plate 11 may be configured to be located, in use, nearer to article 28 to which the sensor 10 is attached than the second plate 12.

The second plate 12 may be configured to be located, in use, nearer to article 28 to which the sensor 10 is attached than the first plate 11.

The sensor 10 may comprise an intermediate layer 20 provided between the first plate 11 and the second plate 12.

The intermediate layer 20 may be an insulating layer and/or a dielectric layer.

The intermediate layer 20 may be or comprise a polymer or a fibreglass layer, or a gas, or air or oil filled polymer pocket or any nonconductive and/or dielectric material. In some embodiments the intermediate layer 20 may be a printed circuit board (PCB).

The intermediate layer 20 may be a substrate to which the first plate and second plate are deposited, etched and/or attached.

The intermediate layer 20 may extend past an edge of the first plate 11 or second plate 12 to provide for a surface or area to which electrical components may be attached.

In some embodiments the sensor or system may comprises one or more wings to provide for a surface or area to which electrical components may be attached.

The electrical components may be housed in one or more housings. The housing may protect the electrical components from the surrounding environments.

The electrical components may be one or more of:
a micro controller and/or processor
a battery or other energy source
a comparator
an analog to digital converter
an integrated component
a resistor
a capacitor
an inductor
a switch
a diode and/or LED
a resonance crystal
an antenna
a transistor
a communications module.
a display module.

The intermediate layer 20 may be less than 25mm in thickness, or between about 0.05 and 25 mm in thickness, or between about 0.05mm and 2.5mm in thickness.

The first plate 11 may be provided on a first side 21 of the sensor. The first side 21 is configured to be located towards the article 28 in use.

The second plate 12 may be provided on a second side 22 of the sensor. The second side 22 may be configured to be located away from the article 28 in use.

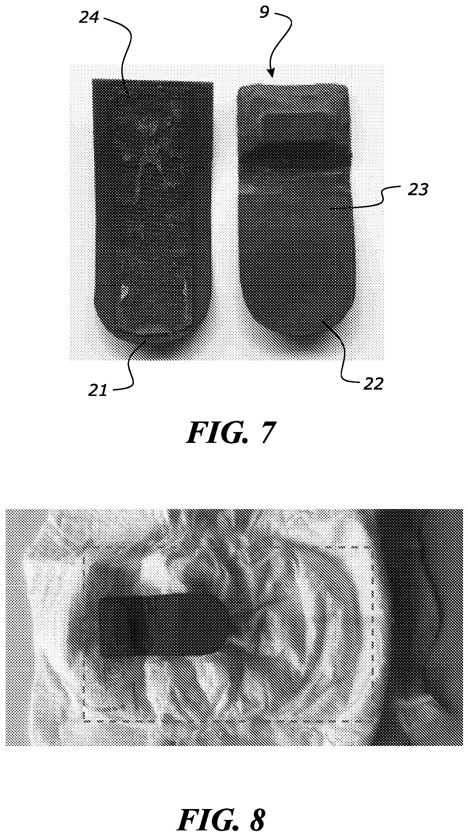

The system and/or sensor 10 may have a protective layer 23 for example as shown in Figs 7 and 8.

The protective layer 23 may be provided as an external layer configured to provide protection of the system and/or sensor.

The protective layer 23 may be configured to encapsulate the system and/or sensor.

The protective layer 23 may be a polymer layer.

The protective layer 23 may be comprise one or more of: paint, or latex paint, or rubber or glass.

The protective layer 23 may be configured to be an electrically insulating material.

The protective layer 23 may be configured to provide for a waterproof covering to stop the ingress of water into the sensor 10.

The protective layer 23 may be configured to be less than about 25mm in thickness, between about 5 and about 20 mm in thickness.

As shown for example in Fig. 7 the sensor 10 may comprise an attachment portion 24.

The attachment portion 24 may be provided on a side and/or a first side of the sensor 10.

The attachment portion 24 may be configured to allow for attachment of the sensor 10 to the article 11. Fig. 8 shows the sensor 10 affixed or attached to a nappy using by the attachment portion 24.

The attachment portion 24 may allow for connection and disconnection of the sensor 10 to and from the article 28. For example, the sensor 10 could be removed and repositioned if required, or removed and transferred to another article.

In some embodiments the sensor 10 may be reused multiple times on various articles 28.

The attachment portion 24 may comprise one or more of:
a hook and/or loop material
an adhesive
a pin or button
an electrostatic attachment mechanism.

In some embodiments, the article 28 may comprise a pocket or feature configured to retain the sensor 10.

The sensor 10 and/or system may be integrally formed with the article 28.

The sensor 10 may comprises at least one isolation layer 25.

The isolation layer 25 may be located on a side and/or the second side 22 of the sensor 10 configured to be located away from the article 28 in use.

The isolation layer 25 may comprise an insulating layer 26.

The insulating layer 26 of the isolation layer 25 may be a polymer layer. In some embodiments, the isolation layer 25 may comprise fiberglass, or glass, or a printed circuit board (PCB), an air, or gas filled pocket.

In some embodiments one or more of the first plate 11 and/or the second plate 12 may be deposited, etched and/or attached to the isolation layer 25.

The isolation layer 25 may comprise a conducting sheet 27.

The conducting sheet 27 may provide for a conductive plane.

The isolation layer 25 may be configured to isolate the sensor from the external environment.

The article may be one or more of: a nappy, a dressing (for example a wound dressing), bedding, clothing or a part of clothing, or cover or container of any kind, an object or material which is configured to take on or absorb moisture, or an object or material which is configured to dry over time (for example concrete, or wood).

In some embodiments, the article may be a wound dressing. When the wound dressing is fitted to cover a wound, it might be difficult to tell whether some liquid, such as blood or pus or other kind of body discharge, or water from outside, is there under the dressing or how much of such liquid is there.

Also disclosed is a system 9 comprising a sensor 10.

Also disclosed is a method for detecting or sensing moisture or wetness of an article to which the system is attached.

The system 9 may detect or sense moisture or wetness of an article to which the system is attached.

The system 9 may comprise a controller or processor.

The system 9 may comprise a sensor 10. The sensor 10 may be the sensor as described above.

The sensor 10 may be configured to store an electrical charge, and wherein the electrical charge storing capacity of the sensor is based on the moisture or wetness of the article.

The controller may be configured to charge the sensor for a first period 50 of time, and after the first period of time 50 discharge the sensor for a second period of time 51, and subsequently measure an output of the sensor. Preferably the output of the sensor is measured at a predetermined measurement time (e.g after discharging time). The predetermined measurement time may vary depending on the expected use of the sensor, and/or expected charge or discharge rate. In some embodiments the predetermined time may be approximately 10-1000 micro seconds after discharging.

The first period of time 50 and/or the second period of time 51 may be predetermined.

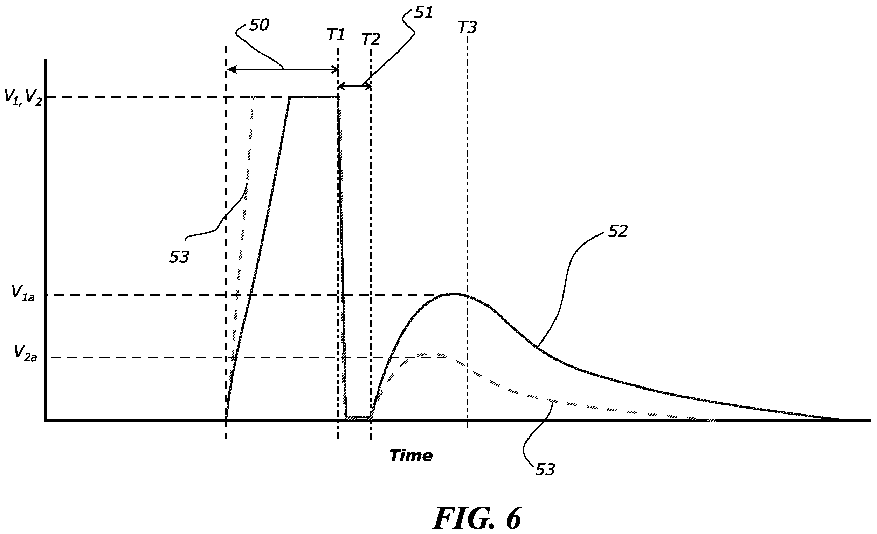

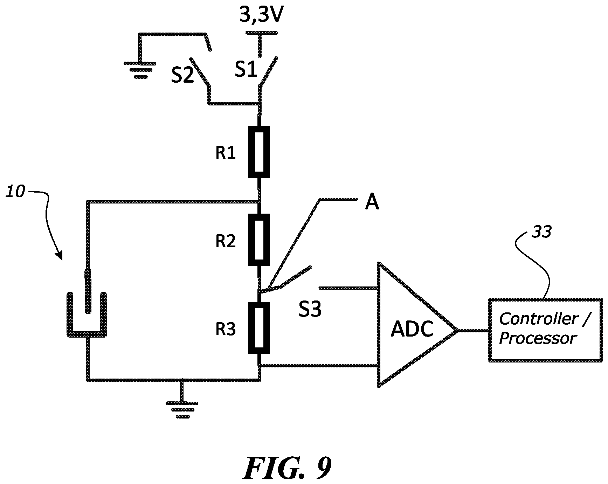

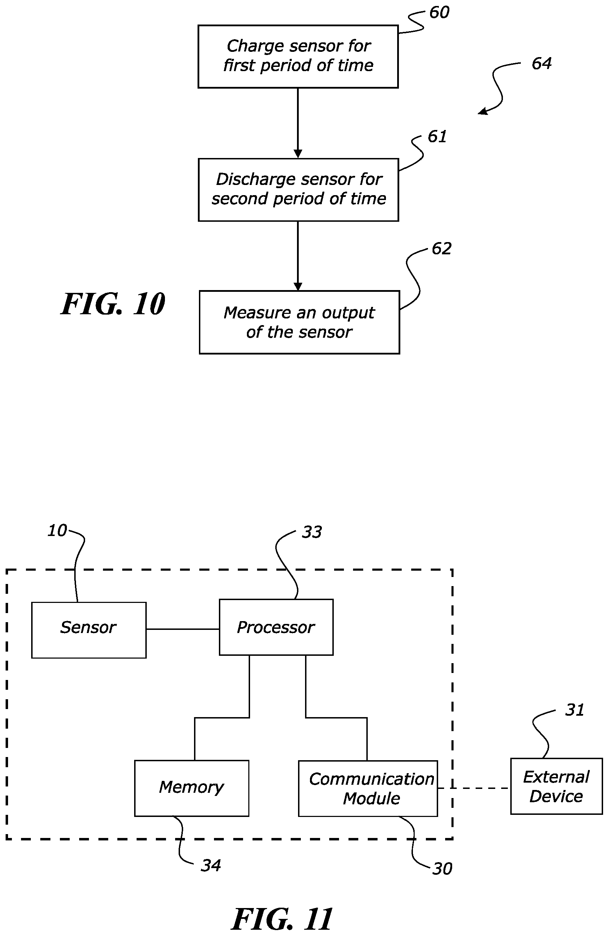

Fig. 6 shows an example of the charge and discharge of the sensor - explained in more detail below, Fig. 9 shows part of an example circuit for the system and Fig. 10 shows an example of a measurement cycle of the sensor.

The measurement cycle 64 contains a charging phase 60, a partial discharge phase 61, and a measurement phase 62.

The measurement cycle 64 may be undertaken in a sequential manner with the charging phase 60 being undertaken first followed by the partial discharge phase 61, and then the measurement phase 62.

Subsequent to the or each measurement cycle 64 the sensor 10 may be allowed to discharge to equilibrium.

The measurement cycle may be undertaken multiple times per second.

At this point an output of the sensor in this case being a voltage across the sensor 10 is V1a or V2a. In the partial discharge phase 61 the sensor 10 is discharged. In some embodiment the switch S2 is closed to introduce an additional discharge path so as to provide for a faster discharge rate. The switch S2 may be opened at the end of the discharge phase 61.

The measurement cycle starts with activating S1. This will charge the sensor (electrochemical cell like) and the parasitic capacitance of the circuit. In the charging phase 60 of the measurement cycle 64 the sensor 10 is charged for a first time period 50 to a time T1. At this point an output of the sensor in this case being a voltage across the sensor 10 is V1 or V2. The sensor 10 is charged by applying a voltage across the sensor 10 for example by closing switch S1 as shown in Fig. 9.

In the discharge or partial discharge phase 61 of the measurement cycle 64 the sensor 10 is discharged for a second period of time 51 from time T1 to time T2. E.g. the source is disconnected and S2 is activated to connect to ground for about 7µs (T2-T1). This will drain the parasitic capacitance in the circuit and invert the electrochemical cell and start the electromotive force to flow back. After this short time of starting to discharge both S1 & S2 are opened to make R1 connected to a high impedance. At the moment S2 becomes high-Z, the ionized environment keeps discharging. This discharge current or EMF will result in a voltage across R3, which is measured by the sensor, preferably at a predetermined time (T3).

When an environment with a better ability to be charged, (a wet nappy for example), more charge will build up and current will flow through R2 and R3 and thus resulting in a higher voltage measured by the ADC which correlates to the wetness of the environment.

The observed behavior is that of a small electrochemical cell. When energy is "injected", the electrolyte (air/diaper/wet diaper) is ionized. A wet diaper has a better ability to get ionized then a dry one. After the charging cycle, the cell changes from a electrolyte cell to a galvanic cell by shorting the circuit for a very small time. Due to the slowness of the cell the EMF starts to flow after this shorting of the circuit which can be measured to indicate the moisture in the environment surrounding the sensor.

In the measurement phase 62 of the measurement cycle 64 an output of the sensor is measured. The output may be based on the amount of charge and/or the amount of charge which has been discharged. In some embodiments the output of the sensor may be a based on an electrical property of the sensor. In some embodiments the output of the sensor may be a based on voltage across the sensor, or another voltage in the circuit, or a current drawn from the sensor (for example through a known resistor. In the circuit of Fig. 9 for example the output is a voltage measured at point A. In Fig. 9 the output is measured using a voltage divider circuit and an analog-to-digital converter. The output of the sensor is then provided to the processor or controller 33.

The processor or controller 33 may, based on the output of the sensor, determine an output indicative of the moisture or wetness of the article based on the measured output of the sensor 10.

As shown in Fig. 6, line 52 (broken line) shows an example measurement cycle 64 for a sensor where moisture or wetness is present, while line 53 shows an example measurement cycle 64 for a sensor where moisture or wetness is not present.

For line 52 where moisture or wetness is present at time T1 the voltage V1 the same as voltage V2 for line 53 where no moisture is present at the sensor 10. The sensor has a larger charge carrying capacity where moisture is present than where no moisture is present. Therefore, given the same charge time (the first period of time 50) and a charge time which ensures the sensor 10 will be fully charged regardless of moisture content, the sensor 10, where moisture is present will hold more charge than the sensor 10 where no moisture is present, however the voltage across the sensor will be the same (i.e. V1=V2).

Subsequently, at the end of the partial discharge phase 61 the output of the sensor 10 voltage V1a for line 52 where moisture or wetness is present will be larger than voltage V2a for line 53 where no moisture is present.

The processor or controller 33 may communicate the output indicative of the moisture or wetness of the article based to an external device 31 via communication module 30.

The communications module 30 may be a wireless communications module such as Bluetooth, Wi-Fi or other suitable RF communication module. It will be apparent that the choice of communication frequency and/or protocol may have advantages applicable to particular use scenarios. For example, relatively low frequency (800 to 900 MHz), may be advantageous in large scale environments such as hospitals, elderly homes and daycare centres where a longer wavelength can penetrate obstacles better.

Protocols such as Bluetooth (BLEv5+, Z-wave and others) may provide mesh technology to communicate over a wider area.

Alternatively, the communications module 30, may be a wired solution such as USB, ethernet, or other protocol. It will be appreciated that a wired protocol may also be used to deliver power to the sensor 10, and/or charge/recharge the sensor.

It will also be appreciated that various inductive power transfer technologies may be useful for charging/recharging an on-board battery.

The sensor 10 may comprise one or more plates. The plates may be electrically conductive. The plates may have any of the features of characteristics of the plates as described above.

The sensor 10 may be charged by providing or applying a voltage or potential difference across the sensor.

The sensor 10 may be charged by providing a voltage or potential difference across a or the first plate and a or the second plate.

The sensor may be charged with a constant voltage

The first period of time may be about 30 µs.

The first period of time may be about 20 µs, or about 10 µs, or about 5 µs, or about 2 µs.

The second period of time may be about 7 µs.

The sensor is discharged through a known resistance.

The output of the sensor is measured by an analog to digital converter and/or via a voltage divider circuit or any other suitable measuring method.

The system may comprises at least one memory element 34.

The system may comprise one or more switches (for example S1 and S2) configured to be controlled by said processor to charge and/or discharge the sensor.

The switches may be any switch known in the art for example one or more transistors.

The output of the sensor may be proportional to the wetness or moisture of the article.

The output of the sensor may be higher when the article is wet or moist than when the article is dry.

The output of the sensor may be lower when the article is dry than when the article is wet or moist.

Fig. 11 shows a diagrammatic overview of the system. The system may comprise one or more communications modules 30. The communication module 30 may be provided as part of the processor or other electrical components.

The communication module 30 may provide for communication between the system and an external device 31. The communication module 30 may provide for communication via a wired, and/or wireless connection.

The communication module 30 may be configured to provide any output of the sensor and/or system.

A display module may also be provided. The display module may display any output of the sensor and/or system.

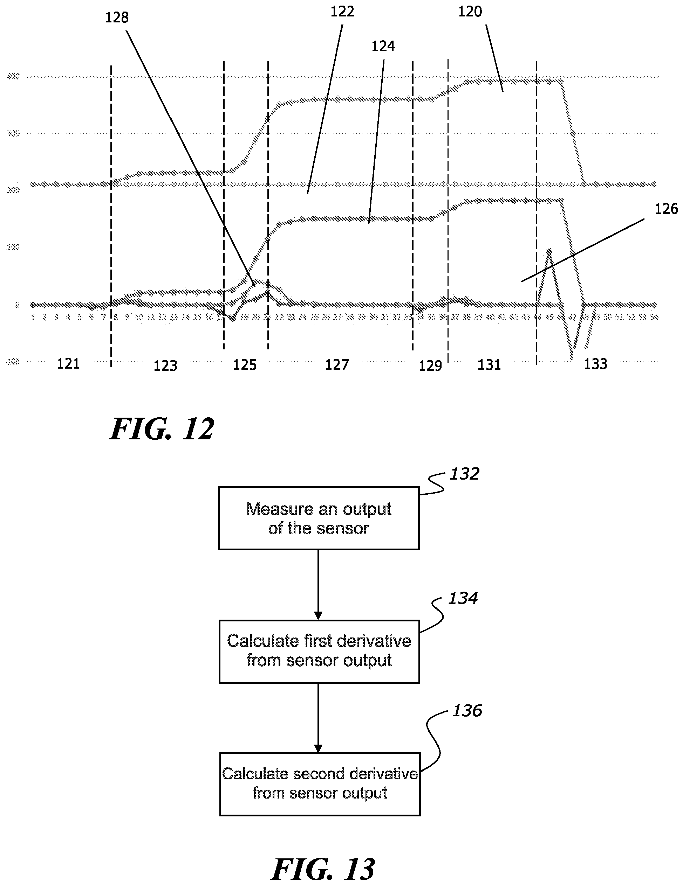

In some embodiments the output indicative of the moisture or wetness and the point of saturation of the article is determined using first and second derivates of the difference between consecutive measurement readings from the sensor, as shown in Fig. 13.

The output indicative of the moisture or wetness and the point of saturation of the article is further based on the comparison between first and second derivatives of the difference between the consecutive measurement readings from the output of the sensor.

In some embodiments, the raw data output from the sensor 10 may be processed on board the sensor.

Alternatively, the raw data from the sensor 10 may be communicated by the communications module 30 to an external device which may further process the data, by calculating for example:
- filtering the data,
- calculating a first derivative,
- calculating a second derivative
- averaging
- comparing any of the above to a predetermined set point
- any other calculation described herein.

The external device may for example be a smart phone, tablet, computer, cloud server, database server or dedicated controller. It will be appreciated that the environment in which the device is intended to be used will at least to some degree dictate preferred configurations. For example, offboard calculation and analysis of the data stream output (wired, or wirelessly) from a sensor 10, may reduce the power requirements of the central module 10 thereby prolonging the expected life from a given battery/charge.

Alternatively, in configurations where a standalone system is preferred, the sensor module 10 may incorporate additional processing steps of the data output and/or may include a user interface in order to enable alerts and/or user interaction to communicate events related to the wetness or moisture of an article etc.

Fig. 12 shows a graph detailing a series of measurement readings from the sensor over time and the corresponding first and second derivatives of these readings.

The determination of the output indicative of the moisture or wetness and the point of saturation of the article based on a comparison between the first and second derivatives will be explained further.

At each period of time, the sensor undertakes a measurement cycle as described in relation to Fig. 6. This measurement cycle is recurrent and independent of the previous cycles. Each measurement cycle produces a reading.

Preferably measurements are taken autonomously, and may occur for example periodically at a predetermined interval, or alternatively still may occur in response to a trigger such as an input from a user or external source.

Each measurement reading is taken by charging and discharging the sensor as shown in and described in relation to Fig. 6. The value obtained at T2 is used as the measurement reading.

Each value obtained at T2 is taken and stored as a measurement reading as an input for analysis. In one embodiment, a number of values are obtained at T2 for each measurement cycle. The number of readings may be averaged to determine the measurement reading for the measurement cycle. This helps to eliminate noise.

Line 120 of Fig. 12 shows an example series of measurement readings over time as described above for a sensor as it progresses through a number of stages (121, 123, 125, 127, 129, 131, 133) of varying moisture or wetness levels of the article, as will be described.

The output indicative of the moisture or wetness of the article is based on an initial base-line measurement or an earlier measurement, as shown by line 122. The initial base-line measurement in this embodiment is the sensor measurement readings taken before the sensor is placed on the article, as shown by region 121 of the graph in Fig. 12.

The base-line measurement or earlier measurement is dependent on the material used to construct the sensor.

In some embodiments, after a measurement reading is taken by the sensor, the base-line measurement or earlier measurement is then removed from this reading, as shown by the readings of line 124 in Fig. 12.

In this embodiment the controller or processor determines a first derivative of the difference between the consecutive measurement readings at each time period, as will be appreciated.

In this embodiment the controller or processor then determines a second derivative of the difference between the consecutive first derivative calculations at each time period, as will be appreciated.

Line 128 shows the series of first derivative calculations of the measurement readings shown by line 124 over time. Line 126 shows the second derivative calculations over time.

In this embodiment each first derivative and second derivative are calculated in real time as the sensor takes measurement readings.

Once calculated, the first and second derivative are compared by the controller or processor. This comparison between the first and the second derivative calculations can be used to determine when of the moisture or wetness of the article is changed and/or when the saturation point of the article has been reached.

In this embodiment the controller or processor compares the current or most up-to-date first and second derivative data points. In some embodiments, the controller or processor also compares the current or most up-to-date first derivative data point to previous first derivative data points, and the current or most up-to-date second derivative data point to previous second derivative data points.

Comparing these data points allows for the different states of varying moisture or wetness, including the saturation point, to be determined. As will be explained by way of example and with reference to Fig. 12 below, the different regions of the graph 121, 123, 125, 127, 129, 131, 133 each indicate an example of a different or varying moisture or wetness level of the sensor or article.

Firstly, no first derivative peak and no second derivative peak indicates that the sensor is in constant or unchanging state of moisture or wetness. This is shown in region 121 where the sensor has not yet been placed on the article, and region 127, where the sensor is reading a constant level of moisture or wetness.

A small first derivative peak and no or almost no second derivative peak indicates that the sensor has been attached to an article. This is shown by way of example in region 123 of Fig. 12.

A large negative first derivative peak and a second derivative peak going from positive to negative indicates removal of the sensor from an article having a high moisture or wetness level. An example of this is shown in region 133.

A positive first derivative peak and second derivative peak going from negative to positive indicates that the moisture or wetness level is increasing. This is shown in region 125 where there is a large moisture or wetness increase.

A slight positive first derivative peak and a second derivative peak going from negative to positive indicates that the article is approaching its saturation point. This is shown at region 129 of Fig. 12.

A decreasing first derivative peak and a decreasing second derivative peak following states indicating a change in moisture or wetness levels such as those described in relation to regions 123, 125 or 129 of Fig. 12 indicates that the article has reached its saturation point.

It will be appreciated that different articles will have different saturation points and the saturation point of each article will be determined by the material of the article.

In some embodiments, once the saturation point of the article has been reached, and this has been determined by the controller or processor, this will trigger a signal. This signal may alert the user to the saturation of the article.

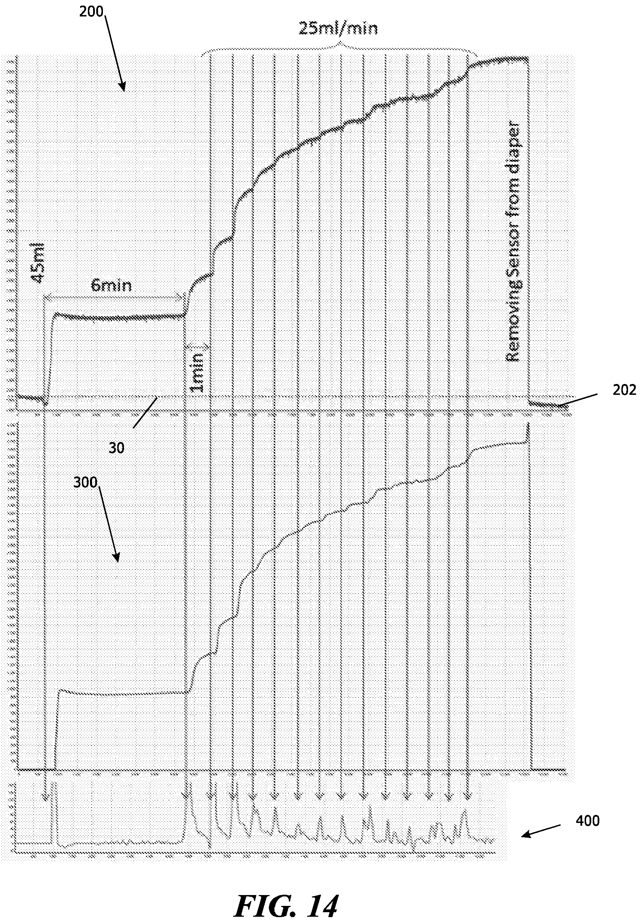

With reference to Fig. 14 test results of a sensor 10 according to the present disclosure will be described. In this test, a sensor 10 was attached to the exterior (dry side) of a nappy, and water was periodically injected into the inside of the nappy. In these tests, the nappy remained stationary throughout the duration of the test.

The test protocol adopted (and shown in Fig. 14) comprised:
step 1 - injecting 45 mL of water over a 14 second time period
step 2 - wait 6 minutes
step 3 - inject 25 mL of water over a 7 second time period
step 4 - wait 1 minute
step 5 - repeat 25 mL injection over a 7 second time period until leakage occurs.

With reference to series 200 shown in Fig. 14, the raw sensor output data is shown. Broken line 201 represents the baseline (with sensor 10 attached to the nappy) before any water is added to the nappy. It can be seen that the final part of the plot 202, drops below the original baseline because the sensor was removed from the nappy.

With reference to series 300 shown in Fig. 14, a filtered plot of the raw data is shown.

The first portion of the plot shows 45 mL of water injected over a 14 second time period. The plot then stabilises for a time period of 6 minutes corresponding to the 6 minute wait, where no further moisture was added.

Subsequent to the 6 minute wait period, a series of one-minute steps are shown within which 25 mL of water were injected.

In the final portion of the plot, the nappy becomes saturated and further injections of water are masked in the sensor output as leakage occurred.

It can be seen from analysing the series 200 and series 300 plots that there is a strong correlation between the volume of water injected and the sensor output sensed by sensor 10. This strong correlation is particularly useful given that the sensor 10 is a non-contact type sensor and was attached to the exterior (dry side) of the nappy.

Further, with reference to series 400 as shown in Fig. 14 a plot of the first derivative of the filtered data is shown. From this plot peaks corresponding to the injection of liquid into the nappy can clearly be seen. Accordingly, the first derivative data can be used to very strongly correlate with and identify an injection event occurring.

Unless the context clearly requires otherwise, throughout the description and the claims, the words "comprise", "comprising", and the like, are to be construed in an inclusive sense as opposed to an exclusive or exhaustive sense, that is to say, in the sense of "including, but not limited to."

Reference to any prior art in this specification is not, and should not be taken as, an acknowledgement or any form of suggestion that that prior art forms part of the common general knowledge in the field of endeavour in any country in the world.

## Claims

1. A system (9) for detecting or sensing moisture or wetness of an article (28) to which the system (9) is attached, the system (9) comprising:
a controller or processor,
a sensor (10), the sensor (10) being configured to store an electrical charge, and wherein the electrical charge storing capacity of the sensor (10) is based on the moisture or wetness of the article (28),
**characterized in that** the controller (33) is configured to:
charge the sensor for a first period of time (50), and after the first period of time (50) partially discharge the sensor (10) for a second period of time (51), and subsequently measure an output of the sensor (10), optionally at a predetermined time,
wherein the controller (33) is configured to determine an output of the sensor (10) indicative of the moisture or wetness of the article (28) based on the measured output of the sensor (10).

2. The system of claim 1, wherein the controller is configured to after the first period of time actively at least partially discharge the sensor for a second period of time.

3. The system of claim 2, wherein the sensor comprise a first and a second plate separated by a dielectric layer (20), and wherein the controller is configured to after the first period of time actively at least partially discharge the sensor by connecting the first and the second plate to substantially the same potential.

4. The system (9) of claim 3, wherein the first and second plate are electrically conductive and when seen in a projection on a plane including the second plate, one of the following conditions is fulfilled:
the projection of the first plate on the plane does not overlap with the second plate; or
the projection of the first plate on the plane overlaps over less than 5% of the surface area of the second plate with the second plate;
wherein the shape of the projection of the first plate on the plane is substantially complementary with the shape of the second plate.

5. The system (9) of claim 4, wherein the first plate is at a distance (d) from the second plate.

6. The system (9) of claim 5, wherein a portion of the second plate (12) is located on each side of the first plate (11).

7. The system (9) of any one of claims 3 to 6, wherein the sensor (10) is charged by providing or applying a voltage or potential difference across a or the first plate (11) and a or the second plate (12).

8. The system (9) of any one of claims 1 to 7, wherein the sensor (10) is charged with a constant voltage.

9. The system (9) of any one of claims 1 to 8, wherein the first period of time (50) is:
a) about 2 µs,
b) about 5 µs,
c) about 10 µs,
d) about 20 µs, or
e) about 30 µs.

10. The system (9) of any one of claims 1 to 9, wherein the second period of time (51) is about 7 µs.

11. The system (9) of any one of claims 1 to 10, wherein the sensor (10) is discharged through a known resistance.

12. The system (9) of any one of claims 1 to 11, wherein the output of the sensor (10) is measured by an analog to digital converter.

13. The system (9) of any one of claims 1 to 12, wherein the system (9) comprises at least one memory element (34).

14. The system (9) of any one of claims 1 to 13, wherein the system (9) comprises one or more switches or transistors configured to be controlled by said processor (33) to charge and/or discharge the sensor (10).

15. The system (9) of any one of claims 1 to 14, wherein the output indicative of the moisture or wetness of the article (28) is further based on an initial base-line measurement.

## Patentansprüche

1. System (9) zum Detektieren oder Abfühlen von Feuchtigkeit oder Nässe eines Artikels (28), an dem das System (9) befestigt ist, wobei das System (9) Folgendes umfasst:
eine Steuerung oder einen Prozessor,
einen Sensor (10), wobei der Sensor (10) ausgelegt ist, um eine elektrische Ladung zu speichern, und wobei die elektrische Ladungs-Speicherkapazität des Sensors (10) auf der Feuchtigkeit oder Nässe des Artikels (28) basiert,
**dadurch gekennzeichnet, dass** die Steuerung (33) ausgelegt ist, um:
den Sensor über eine erste Zeitspanne (50) zu laden und nach der ersten Zeitspanne (50) den Sensor (10) über eine zweite Zeitspanne (51) teilweise zu entladen und anschließend, gegebenenfalls zu einem vorbestimmten Zeitpunkt, ein Ausgangssignal des Sensors (10) zu messen,
wobei die Steuerung (33) ausgelegt ist, um ein Ausgangssignal des Sensors (10) zu bestimmen, das indikativ für die Feuchtigkeit oder Nässe des Artikels (28) basierend auf dem gemessenen Ausgangssignal des Sensors (10) ist.

2. System nach Anspruch 1, wobei die Steuerung ausgelegt ist, um den Sensor nach der ersten Zeitspanne über eine zweite Zeitspanne aktiv zumindest teilweise zu entladen.

3. System nach Anspruch 2, wobei der Sensor eine erste und eine zweite Platte umfasst, die durch eine dielektrische Schicht (20) getrennt sind, und wobei die Steuerung ausgelegt ist, um den Sensor nach der ersten Zeitspanne durch Verbinden der ersten und zweiten Platte mit dem im Wesentlichen gleichen Potenzial aktiv zumindest teilweise zu entladen.

4. System (9) nach Anspruch 3, wobei die erste und die zweite Platte elektrisch leitend sind und bei Betrachtung in einer Projektion auf eine Ebene, die die zweite Platte beinhaltet, eine der folgenden Bedingungen erfüllt ist:
die Projektion der ersten Platte auf die Ebene überlappt nicht mit der zweiten Platte; oder
die Projektion der ersten Platte auf die Ebene überlappt zu weniger als 5 % der Oberfläche der zweiten Platte mit der zweiten Platte;
wobei die Form der Projektion der ersten Platte auf die Ebene im Wesentlichen zu der Form der zweiten Platte komplementär ist.

5. System (9) nach Anspruch 4, wobei sich die erste Platte in einem Abstand (d) von der zweiten Platte befindet.

6. System (9) nach Anspruch 5, wobei ein Abschnitt der zweiten Platte (12) auf jeder Seite der ersten Platte (11) angeordnet ist.

7. System (9) nach einem der Ansprüche 3 bis 6, wobei der Sensor (10) durch Bereitstellen oder Anlegen einer Spannungs- oder Potenzialdifferenz an eine oder die erste Platte (11) und eine oder die zweite Platte (12) geladen wird.

8. System (9) nach einem der Ansprüche 1 bis 7, wobei der Sensor (10) mit einer konstanten Spannung geladen wird.

9. System (9) nach einem der Ansprüche 1 bis 8, wobei die erste Zeitspanne (50)
a) etwa 2 µs,
b) etwa 5 µs,
c) etwa 10 µs,
d) etwa 20 µs oder
e) etwa 30 µs lang ist.

10. System (9) nach einem der Ansprüche 1 bis 9, wobei die zweite Zeitspanne (51) etwa 7 µs lang ist.

11. System (9) nach einem der Ansprüche 1 bis 10, wobei der Sensor (10) durch einen bekannten Widerstand entladen wird.

12. System (9) nach einem der Ansprüche 1 bis 11, wobei das Ausgangssignal des Sensors (10) von einem Analog-Digital-Umsetzer gemessen wird.

13. System (9) nach einem der Ansprüche 1 bis 12, wobei das System (9) zumindest ein Speicherelement (34) umfasst.

14. System (9) nach einem der Ansprüche 1 bis 13, wobei das System (9) einen oder mehrere Schalter oder Transistoren umfasst, die ausgelegt sind, um von dem Prozessor (33) gesteuert zu werden, um den Sensor (10) zu laden und/oder zu entladen.

15. System (9) nach einem der Ansprüche 1 bis 14, wobei das Ausgangssignal, das indikativ für die Feuchtigkeit oder Nässe des Artikels (28) ist, außerdem auf einer anfänglichen Basislinienmessung basiert.

## Revendications

1. Système (9) pour détecter ou capter la teneur en eau ou l'humidité d'un article (28) auquel le système (9) est fixé, le système (9) comprenant :
un dispositif de commande ou un processeur,
un capteur (10), le capteur (10) étant configuré pour stocker une charge électrique, et dans lequel la capacité de stockage de charge électrique du capteur (10) est basée sur la teneur en eau ou l'humidité de l'article (28),
**caractérisé en ce que** la dispositif de commande (33) est configuré pour :
charger le capteur pendant une première période de temps (50), et après la première période de temps (50) décharger partiellement le capteur (10) pendant une seconde période de temps (51), et mesurer ensuite une sortie du capteur (10), facultativement à un temps prédéterminé,
dans lequel le dispositif de commande (33) est configuré pour déterminer une sortie du capteur (10) indiquant la teneur en eau ou l'humidité de l'article (28) sur la base de la sortie mesurée du capteur (10).

2. Système selon la revendication 1, dans lequel le dispositif de commande est configuré pour, après la première période de temps, décharger activement au moins partiellement le capteur pendant une seconde période de temps.

3. Système selon la revendication 2, dans lequel le capteur comprend une première et une seconde plaque séparées par une couche diélectrique (20), et dans lequel le dispositif de commande est configuré pour, après la première période de temps, décharger activement au moins partiellement le capteur en connectant la première et la seconde plaque sensiblement au même potentiel.

4. Système (9) selon la revendication 3, dans lequel les première et seconde plaques sont électriquement conductrices et, lorsqu'elles sont vues dans une projection sur un plan comprenant la seconde plaque, l'une des conditions suivantes est remplie :
la projection de la première plaque sur le plan ne chevauche pas la seconde plaque ; ou
la projection de la première plaque sur le plan chevauche moins de 5 % de l'aire de surface de la seconde plaque avec la seconde plaque ;
dans lequel la forme de la projection de la première plaque sur le plan est sensiblement complémentaire de la forme de la seconde plaque.

5. Système (9) selon la revendication 4, dans lequel la première plaque est à une distance (d) de la seconde plaque.

6. Système (9) selon la revendication 5, dans lequel une partie de la seconde plaque (12) est située de chaque côté de la première plaque (11).

7. Système (9) selon l'une quelconque des revendications 3 à 6, dans lequel le capteur (10) est chargé en fournissant ou en appliquant une tension ou une différence de potentiel entre une ou la première plaque (11) et une ou la seconde plaque (12).

8. Système (9) selon l'une quelconque des revendications 1 à 7, dans lequel le capteur (10) est chargé avec une tension constante.

9. Système (9) selon l'une quelconque des revendications 1 à 8 dans lequel la première période de temps (50) est :
a) d'environ 2 µs,
b) d'environ 5 µs,
c) d'environ 10 µs,
d) d'environ 20 µs, ou
e) d'environ 30 µs.

10. Système (9) l'une quelconque des revendications 1 à 9, dans lequel la seconde période de temps (51) est d'environ 7 µs.

11. Système (9) selon l'une quelconque des revendications 1 à 10, dans lequel le capteur (10) est déchargé via une résistance connue.

12. Système (9) selon l'une quelconque des revendications 1 à 11, dans lequel la sortie du capteur (10) est mesurée par un convertisseur analogique-numérique.

13. Système (9) selon l'une quelconque des revendications 1 à 12, dans lequel le système (9) comprend au moins un élément de mémoire (34).

14. Système (9) selon l'une quelconque des revendications 1 à 13, dans lequel le système (9) comprend un ou plusieurs commutateurs ou transistors configurés pour être commandés par ledit processeur (33) pour charger et/ou décharger le capteur (10).

15. Système (9) selon l'une quelconque des revendications 1 à 14, dans lequel la sortie indicative de la teneur en eau ou de l'humidité de l'article (28) est en outre basée sur une mesure de ligne de base initiale.
